# EUROPEAN PATENT APPLICATION

(11) **EP 4 437 980 A1**
(43) Date of publication of application: **02.10.2024**
(21) Application number: 23165296.7
(22) Date of filing: 30.03.2023
(51) Int. Cl.: A61B 17/32, A61B 17/3205, A61B 18/24, A61B 17/22, A61B 17/00, A61B 18/00

(54) **SHEATH AND BALLOON CATHETER ASSEMBLY FOR AORTIC VALVE LEAFLET REMOVAL AND ASSOCIATED DEVICES, SYSTEMS, AND METHODS**

(71) Applicant: Koninklijke Philips N.V., 5656 AG Eindhoven (NL)
(72) Inventor: MORRILL, Meghan B., Eindhoven (NL); ANDERSON, Michael, Eindhoven (NL); FRANCIS, Nathan C., 5656 AG Eindhoven (NL); GRACE, Kenneth Peter, Eindhoven (NL)
(74) Representative: Philips Intellectual Property & Standards

(57) **Abstract**

A system includes an outer sheath, an inner sheath, and a catheter. The outer sheath can be positioned within a heart. The outer sheath includes a lumen and a cutting tip. The inner sheath is positioned within the lumen of the outer sheath, the inner sheath includes a lumen. The catheter is positioned within the lumen of the inner sheath. The catheter includes a balloon. In the balloon's unexpanded state, the catheter and the inner sheath can receive an aortic valve leaflet within a gap between the balloon and the inner portion of the inner sheath. In the balloon's expanded state, the catheter and the inner sheath can capture the leaflet within the gap. The cutting tip of the outer sheath can cut the leaflet while the leaflet is captured such that a cut portion is captured within the gap for removal from the patient.

## Description

### TECHNICAL FIELD

The subject matter described herein relates to cardiac valve replacement and, in particular, to the reducing or eliminating blood flow obstruction to the human heart during and after cardiac valve replacement. For example, a sheath and balloon catheter assembly may be utilized for aortic valve leaflet removal of a stenosed and/or otherwise degenerated natural aortic valve or a stenosed and/or otherwise degenerated replacement aortic valve that may obstruct blood flow to the human heart during an aortic valve replacement procedure.

### BACKGROUND

One common type of valve disease is aortic valve stenosis and, with an advent of minimally invasive procedures, valve replacement is most common therapy. Transvenous/transcatheter aortic valve replacement (TAVR) is becoming more common of a procedure as technology and doctor skillsets improve. This minimally invasive approach to valve replacement is an alternative to open heart surgical aortic valve replacement (SAVR). One difference between a TAVR procedure and a SAVR procedure is, in the SAVR procedure, the natural aortic valve is removed during an open-heart procedure that is performed once, i.e. when the natural aortic valve is replaced. In a TAVR procedure, the damaged valve, whether the valve is a natural aortic valve or a previous SAVR or TAVR valve, is left in place. These valves may have anatomical abnormalities, calcification, or infection. Inserting a new valve over the previous valve may cause complications in the TAVR procedure, including valve migration, valve embolization, paravalvular leakage, patient-prosthesis mismatch, and blockage of the coronary arteries restricting blood flow to the heart.

TAVR has been approved for low-risk patients, which in general are of younger age and live longer. Recent studies have shown that the life of a TAVR valve will only be on average 8 years, so there will be an increase in replacement TAVR procedures. Old TAVR leaflets may be fibrosed, calcified, or thickened during its life creating a barrier to replacement valve. The old leaflets also pose a more serious risk of coronary obstruction than native valves.

The information included in this Introduction section of the specification, including any references cited herein and any description or discussion thereof, is included for context and/or technical reference purposes only and is not to be regarded as subject matter by which the scope of the disclosure is to be bound or otherwise limited in any manner.

### SUMMARY

Disclosed are intraluminal devices, systems, and methods that assist in the removal of old valve leaflets, whether natural aortic leaflets or previous TAVR leaflets, and prepare site for valve replacement during a cardiac valve replacement. In some instances, deployment of a new TAVR valve on top of an existing damaged natural aortic valve or previous TAVR valve may lead to complications in the cardiac valve replacement procedure. Removing old valve leaflets helps prepare the implant site for a cleaner valve deployment and operation. Removal of the leaflets may introduce issues with aortic regurgitation or aortic insufficiency in the patient. This is especially important in valve-in-valve TAVR procedure as the risk for coronary obstruction is higher. During the implantation of a replacement valve during a TAVR procedure, the action of expanding the replacement valve may push the current leaflets to the sides of the aorta and cover the openings to the coronary arteries, reducing oxygenated blood to the heart. This is a critical risk during the valve replacement, so by utilizing a laser sheath and balloon catheter assembly device, parts of the aortic valve leaflets may be removed prior to expanding a replacement valve.

In some aspects, in order to remove parts of the aortic valve leaflets prior to expanding a replacement valve, an inner sheath and balloon catheter are utilized to capture one or more leaflets between an outer surface of the balloon and an inner surface of the inner sheath. A cutting feature on the outer sheath is then utilized to cut the one or more leaflets while the one or more leaflets are received within the gap such that a cut portion of the leaflet is captured within the gap for removal from the patient.

In an exemplary aspect, a system is provided. The system includes an outer sheath configured to be positioned within a heart of a patient, wherein the outer sheath comprises a proximal portion, a distal portion, a lumen, and a cutting tip positioned at the distal portion; an inner sheath configured to be positioned within the lumen of the outer sheath, wherein the inner sheath comprises a lumen and an inner diameter; a catheter configured to positioned within the lumen of the inner sheath, wherein the catheter comprises a proximal portion, a distal portion, and a balloon positioned at the distal portion, wherein the balloon comprises an expanded state and an unexpanded state; wherein, in the unexpanded state of the balloon, the catheter and the inner sheath are configured to receive a leaflet of an aortic valve within a gap between the balloon and the inner diameter of the inner sheath; wherein, in the expanded state of the balloon, the catheter and the inner sheath are configured to capture the leaflet within the gap; and wherein the cutting tip of the outer sheath is configured to cut the leaflet while the leaflet is captured within the gap such that a cut portion of the leaflet is captured within the gap for removal from the patient.

In some aspects, the inner sheath comprises a proximal portion, a distal portion, a perimeter, and a plurality of tabs positioned at the distal portion, the plurality of tabs are spaced from one another around the perimeter of the inner sheath, and the plurality of tabs are configured to be positioned within sinuses of the aortic valve. In some aspects, the catheter and the inner sheath are configured to receive the leaflet and capture the leaflet between the balloon and a plurality of tabs. In some aspects, the system includes a pump configured to transition the balloon between expanded state and the unexpanded state. In some aspects, the inner sheath comprises an outer surface with a hydrophilic coating. In some aspects, the inner sheath comprises a proximal portion and a distal portion, and at least one of the proximal portion of the inner sheath or the proximal portion of the outer sheath comprises a hemostasis valve. In some aspects, the inner sheath comprises a proximal portion and a distal portion, and at least one of the distal portion of the inner sheath or the distal portion of the outer sheath comprises a radiopaque marker. In some aspects, the inner sheath comprises a proximal portion and a distal portion, and at least one of the proximal portion of the inner sheath or the proximal portion of the outer sheath comprises a visible marker. In some aspects, the cutting tip comprises an optical fiber.

In an exemplary aspect, a method is provided. The method includes moving a catheter within an aorta and through an aortic valve such that a distal portion of the catheter is positioned within a left ventricle of a heart, wherein the catheter comprises a balloon positioned at the distal portion of the catheter, wherein the balloon comprises an expanded state and an unexpanded state; moving an inner sheath within the aorta and along the catheter such the catheter is positioned within a lumen of the inner sheath; receiving a leaflet of the aortic valve within a gap between the balloon and an inner diameter of the inner sheath with the balloon is in the unexpanded state; capturing the leaflet within a gap with the balloon is in the expanded state; moving an outer sheath within the aorta and along the inner sheath such the inner sheath is positioned within a lumen of the outer sheath, wherein the outer sheath comprises a distal portion and a cutting tip positioned at the distal portion of the outer sheath; cutting, with the cutting tip, the leaflet while the leaflet is captured within the gap such that a cut portion of the leaflet is captured within the gap; and removing the cut portion of the leaflet from a body of a patient.

In some aspects, the method includes transitioning the balloon from the unexpanded state to the expanded state while the leaflet is received within the gap. In some aspects, the inner sheath comprises a proximal portion, a distal portion, a perimeter, and a plurality of tabs positioned at the distal portion, the plurality of tabs are spaced from one another around the perimeter of the inner sheath, and moving the inner sheath comprises positioning the plurality of tabs within sinuses of the aortic valve. In some aspects, the leaflet is received and captured between the balloon and a plurality of tabs. In some aspects, the distal portion of the catheter is positioned within the left ventricle such that: the leaflet contacts the catheter during diastole to prevent mitral regurgitation; and the leaflet is spaced from the catheter during systole to allow blood flow from the left ventricle to the aorta. In some aspects, receiving the leaflet comprises moving a distal portion of the inner sheath distally towards the distal portion of the catheter. In some aspects, cutting the leaflet comprises moving the distal portion of the outer sheath distally towards the distal portion of the catheter such that the distal portion of the outer sheath is positioned within the left ventricle. In some aspects, the distal portion of the outer sheath is positioned within the left ventricle such that: the leaflet contacts the outer sheath during diastole to prevent mitral regurgitation; and the leaflet is spaced from the outer sheath during systole to allow blood flow from the left ventricle to the aorta. In some aspects, the method includes moving a valve delivery catheter through the lumen of the outer sheath, and the valve delivery catheter comprises a transcatheter aortic valve replacement (TAVR) valve removably coupled at a distal portion of the valve delivery catheter.

This Summary is provided to introduce a selection of concepts in a simplified form that are further described below in the Detailed Description. This Summary is not intended to identify key features or essential features of the claimed subject matter, nor is it intended to limit the scope of the claimed subject matter. A more extensive presentation of features, details, utilities, and advantages of aspects of the present disclosure, e.g., as defined in the claims, is provided in the following written description of various examples and/or aspects of the disclosure and illustrated in the accompanying drawings.

### BRIEF DESCRIPTION OF THE DRAWINGS

Illustrative aspects of the present disclosure will be described with reference to the accompanying drawings, of which:
Fig. 1A illustrates a side view of a human heart according to aspects of the present disclosure.
Fig. 1B is a cross-sectional side view of a human heart according to aspects of the present disclosure.
Fig. 2 is a cross-sectional side view of an aortic valve replacement in a human heart according to aspects of the present disclosure.
Fig. 3A is a diagrammatic cross-sectional top view of an aortic valve replacement of a degenerated natural aortic valve utilizing a TAVR valve, according to aspects of the present disclosure.
Fig. 3B is a diagrammatic cross-sectional top view of a valve-in-valve replacement of a degenerated TAVR valve utilizing a new TAVR valve, according to aspects of the present disclosure.
Fig. 3C is a diagrammatic cross-sectional top view of a valve-in-valve replacement of a degenerated surgical aortic valve replacement (SAVR) valve utilizing a new TAVR valve, according to aspects of the present disclosure.
Fig. 4A is a diagrammatic top view of an aortic valve, according to aspects of the present disclosure.
Fig. 4B is a diagrammatic cross-sectional side view of a TAVR implant in an aortic valve according to aspects of the present disclosure.
Fig. 5 is diagrammatic, schematic view of a system according to aspects of the present disclosure.
Fig. 6 is a schematic diagram of a processing system according to aspects of the present disclosure.
Fig. 7 is a diagrammatic cross-sectional top-view of an intraluminal device according to aspects of the present disclosure.
Figs. 8A and 8B illustrate a diagrammatic cross-sectional view of a balloon catheter according to aspects of the present disclosure.
Fig. 9 is a diagrammatic cross-sectional top-view of an inner sheath according to aspects of the present disclosure.
Fig. 10 is a diagrammatic cross-sectional top-view of an outer/cutting sheath according to aspects of the present disclosure.
Figs. 11A and 11B are diagrammatic views of an intraluminal device according to aspects of the present disclosure.
Figs. 12A and 12B are diagrammatic cross-sectional side views of an aortic valve according to aspects of the present disclosure.
Fig. 13 is a diagrammatic cross-sectional side views of an aortic valve according to aspects of the present disclosure.
Fig. 14 is diagrammatic view of an intraluminal device inserted into an aortic valve of a human heart according to aspects of the present disclosure.
Fig. 15 is diagrammatic view of an inner sheath positioned in an aortic valve of a human heart according to aspects of the present disclosure.
Fig. 16 is a diagrammatic cross-sectional side views of an aortic valve according to aspects of the present disclosure.
Fig. 17 is diagrammatic view of aortic valve leaflets of an aortic valve being secured between an outer surface of a balloon in an inflated state and an inside of the inner sheath according to aspects of the present disclosure.
Fig. 18 is a diagrammatic cross-sectional side view of an aortic valve according to aspects of the present disclosure.
Fig. 19 is diagrammatic view of an intraluminal device inserted into an aortic valve of a human heart according to aspects of the present disclosure.
Fig. 20 is diagrammatic view of an intraluminal device inserted into an aortic valve of a human heart according to aspects of the present disclosure.
Fig. 21 is diagrammatic view of an intraluminal device inserted into an aortic valve of a human heart according to aspects of the present disclosure.
Fig. 22A is a diagrammatic cross-sectional top-view of an intraluminal device according to aspects of the present disclosure.
Fig. 22B illustrates a cross-sectional view of the intraluminal device as seen along the lines of the section A-A of Fig. 22A taken therein, according to aspects of the present disclosure.
Fig. 23A is a diagrammatic cross-sectional side view of an aortic valve, during systole, according to aspects of the present disclosure.
Fig. 23B is a diagrammatic cross-sectional side view of an aortic valve, during diastole, according to aspects of the present disclosure.
Fig. 24 is a diagrammatic cross-sectional side view of an aortic valve according to aspects of the present disclosure.
Fig. 25 is a flow diagram of the process performed in removing one or more portions of leaflets of a degenerated natural aortic valve or a degenerated replacement aortic valve according to aspects of the present disclosure.

### DETAILED DESCRIPTION

For the purposes of promoting an understanding of the principles of the present disclosure, reference will now be made to the examples illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one example and/or aspect may be combined with the features, components, and/or steps described with respect to other examples and/or aspects of the present disclosure. Additionally, while the description below may refer to blood vessels, it will be understood that the present disclosure is not limited to such applications. For example, the devices, systems, and methods described herein may be used in any body chamber or body lumen, including an esophagus, veins, arteries, intestines, ventricles, atria, or any other body lumen and/or chamber. For the sake of brevity, however, the numerous iterations of these combinations will not be described separately.

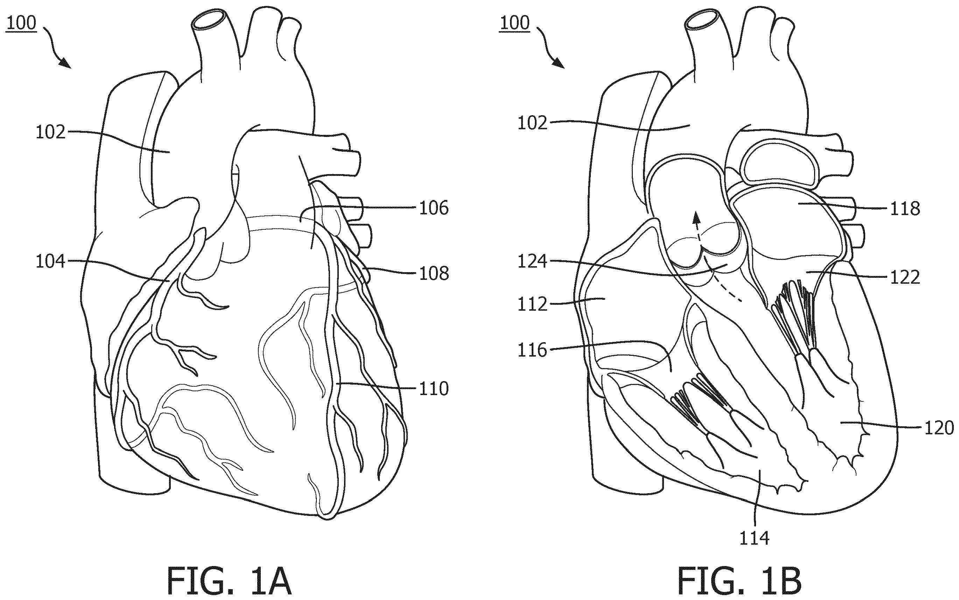

Referring to Fig. 1A, shown is a side view of a human heart 100 according to aspects of the present disclosure. Visible are an aorta 102 from which stems a right coronary artery 104 and a left main coronary artery 106. The left main coronary artery 106 branches into a left circumflex coronary artery 108 and a left anterior descending coronary artery 110. The right coronary artery 104, the left main coronary artery 106, the left circumflex coronary artery 108, and the left anterior descending coronary artery 110 are the arteries that provide oxygen-rich blood to muscles of the human heart 100.

Fig. 1B is a cross-sectional side view of a human heart 100 according to aspects of the present disclosure. Visible are a right atrium 112 and a right ventricle 114. In that regard, oxygen-poor blood enters the human heart 100 in the right atrium 112 and travels to the right ventricle 114 through the tricuspid valve 116. The oxygen-poor blood leaves the right ventricle 114 and travels to the lungs. Also visible are a left atrium 118 and a left ventricle 120. In that regard, oxygen-rich blood is received from the lungs in the left atrium 118 and travels to the left ventricle 120 through the mitral valve 122. The oxygen-rich blood leaves the left ventricle 120 and goes out to the body through the aorta 102 via an aortic valve 124.

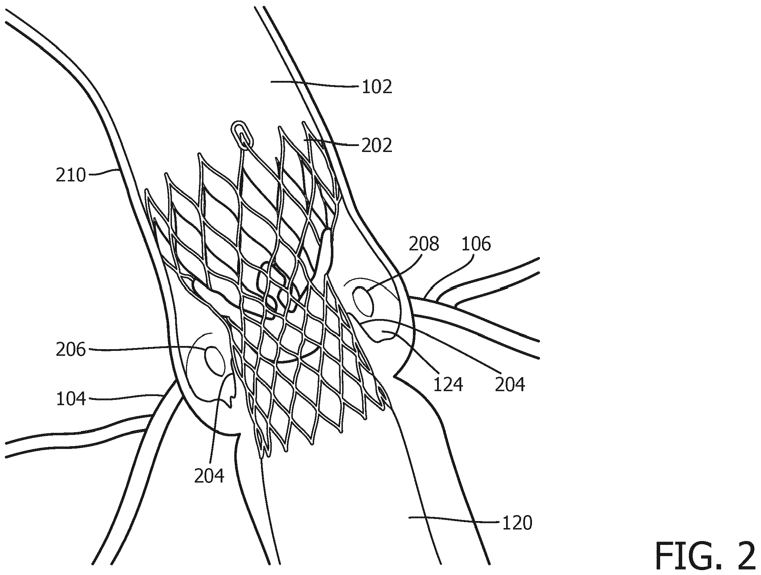

Fig. 2 is a cross-sectional side view of an aortic valve 124 replacement in a human heart 100 according to aspects of the present disclosure. In some aspects, e.g., when aortic valve stenosis has occurred to the aortic valve 124 that keeps blood flowing in the correct direction from the left ventricle 120 to the aorta 102, a transvenous/transcatheter aortic valve repair (TAVR) procedure may be performed to replace the natural aortic valve 124 with a replacement aortic valve 202. In some instances, portions of one or more leaflets 204 of the natural aortic valve 124 may be resected so that openings 206 and 208 to the right coronary artery 104 and the left main coronary artery 106, respectively, so that, when the one or more leaflets 204 are pressed against the natural heart wall 210, the openings 206 and 208 remain unobstructed so that oxygen-rich blood may flow to the muscles of the human heart. The natural heart wall 210 may be a natural aorta wall, a natural heart chamber wall, or a natural aortic valve wall.

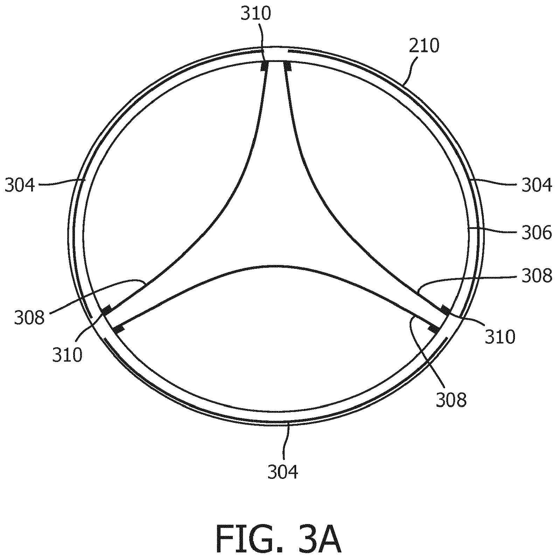

Fig. 3A is a diagrammatic cross-sectional top view of an aortic valve replacement of a degenerated natural aortic valve utilizing a TAVR valve, according to aspects of the present disclosure. Visible are the natural heart wall 210, the natural aortic valve leaflets 304, the TAVR valve wall 306, the TAVR valve leaflets 308, and the TAVR commissural tabs 310. The TAVR valve leaflets 308 are constructed from bovine animal tissue that are coupled to the wire frame of the TAVR valve wall 306 via the commissural tabs 310. When inserted, the TAVR valve pushes the remaining unresected portions of the natural aortic valve leaflets 304 against the natural heart wall 210 such that the natural aortic valve leaflets 304 are pinned and/or secured between an outside of the TAVR valve wall 306 and the natural heart wall 210.

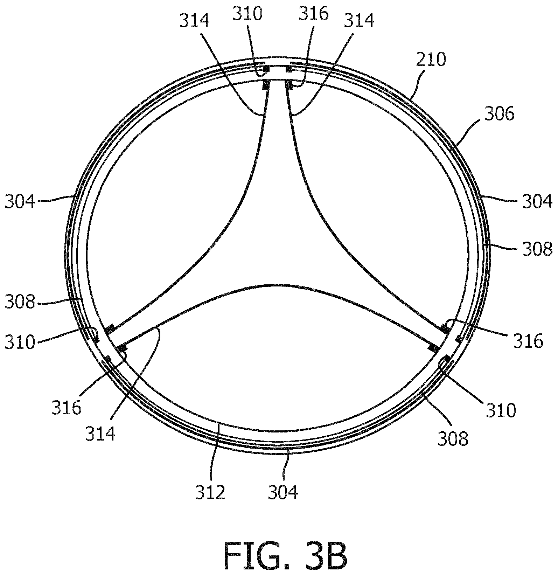

Fig. 3B is a diagrammatic cross-sectional top view of a valve-in-valve replacement of a degenerated TAVR valve utilizing a new TAVR valve, according to aspects of the present disclosure. Visible are the natural heart 210, the natural aortic valve leaflets 304, the degenerated TAVR valve wall 306, the degenerated TAVR valve leaflets 308, the degenerated TAVR commissural tabs 310, the new TAVR valve wall 312, the new TAVR valve leaflets 314, and the new TAVR commissural tabs 316. The new TAVR valve leaflets 314 are constructed from bovine animal tissue that are coupled to the wire frame of the new TAVR valve wall 312 via the commissural tabs 316. When inserted, the TAVR valve pushes the remaining unresected portions of the degenerated TAVR valve leaflets 308 against an inside of the degenerated TAVR valve wall 306 such that the degenerated TAVR valve leaflets 308 are pinned and/or secured between an outside of the new TAVR valve wall 312 and the inside wall of the degenerated TAVR valve wall 306.

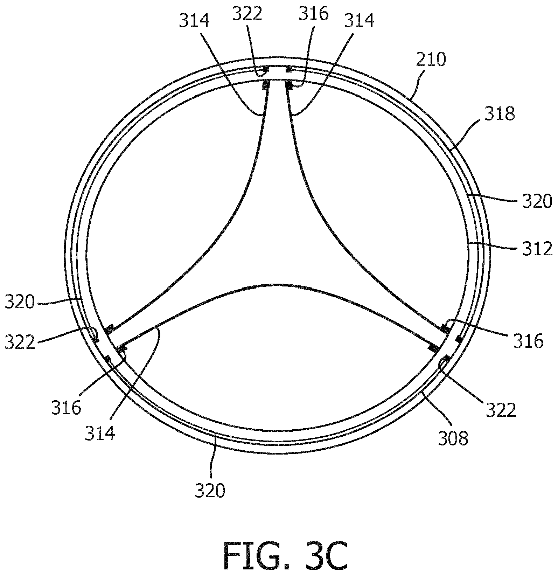

Fig. 3C is a diagrammatic cross-sectional top view of a valve-in-valve replacement of a degenerated surgical aortic valve replacement (SAVR) valve utilizing a new TAVR valve, according to aspects of the present disclosure. Visible are the natural heart wall 210, a degenerated SAVR valve wall 318, the degenerated SAVR valve leaflets 320, the degenerated SAVR commissural tabs 322, the new TAVR valve wall 312, the new TAVR valve leaflets 314, and the new TAVR commissural tabs 316. The new TAVR valve leaflets 314 constructed from bovine animal tissue are coupled to the wire frame of the new TAVR valve wall 312 via the commissural tabs 316. When inserted, the TAVR valve pushes the remaining unresected portions of the degenerated SAVR valve leaflets 320 against an inside of the degenerated SAVR valve wall 318 such that the degenerated TAVR valve leaflets 320 are pinned and/or secured between an outside of the new TAVR valve wall 312 and the inside wall of the degenerated SAVR valve wall 318.

The natural valve, TAVR valve, and/or SAVR valve can be degenerated (e.g., does not function as it should) as a result of various causes. For example, the natural valve, TAVR valve, and/or SAVR valve can be degenerated because it is stenosed, because it is regurgitant, etc.

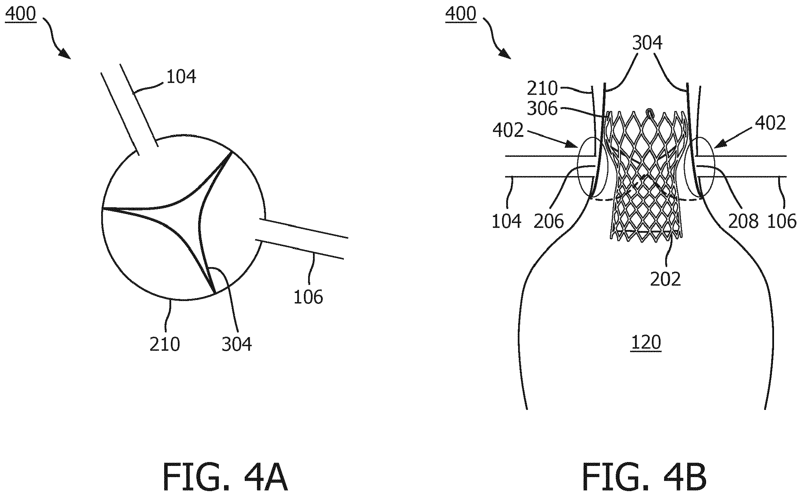

Fig. 4A is a diagrammatic top view of an aortic valve 400, according to aspects of the present disclosure. Visible are the natural heart wall 210, the aortic valve leaflets 304, the right coronary artery 104, and the left main coronary artery 106. Fig. 4B is a diagrammatic cross-sectional side view of a TAVR implant in an aortic valve 400 according to aspects of the present disclosure. Visible are the natural heart wall 210, the aortic valve leaflets 304, the right coronary artery 104, and the left main coronary artery 106. In some aspects, when a replacement aortic valve 202 is implanted and the aortic valve leaflets 304 are not resected or insufficiently resected, the aortic valve leaflets 304, when pinned and/or secured between an outside of the TAVR valve wall 306 and the natural heart wall 210, obstruct the openings 206 and 208 to the right coronary artery 104 and the left main coronary artery 106, respectively, as indicated by areas 402.

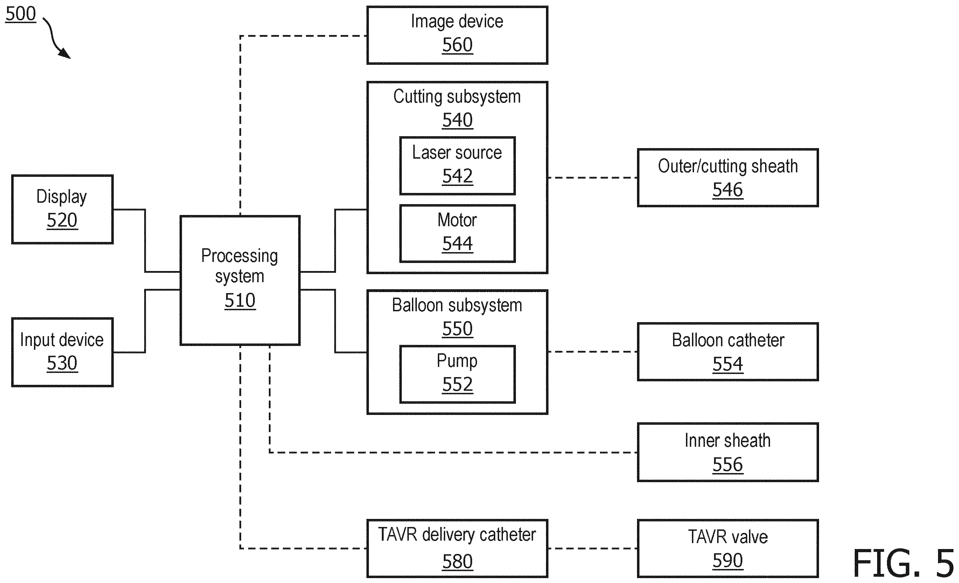

Fig. 5 is diagrammatic, schematic view of a system 500 according to aspects of the present disclosure. The system 500 may be configured to evaluate (e.g., assess), display, and/or control (e.g., modify) one or more aspects of a cardiac valve replacement. For instance, the system 500 may be utilized to monitor and/or control one or more portions of a cardiac valve replacement process while reducing and/or eliminating aortic regurgitation, as described in greater detail below. In this regard, the system 500 may be used to assess coronary vessels and/or heart tissue (e.g., the myocardium). As illustrated, the system 500 may include a processing system 510 in communication with a display device 520 (e.g., an electronic display or monitor), an input device 530 (e.g., a user input device, such as a keyboard, mouse, joystick, microphone, and/or other controller or input device), a cutting subsystem 540, a balloon subsystem 550, and/or an imaging device 560. As illustrated, the system 500 may further include a TAVR delivery catheter 580 and a TAVR valve 590, which may be completely mechanical or connected to processing system 510 in order to provide intraluminal data to processing system 510.

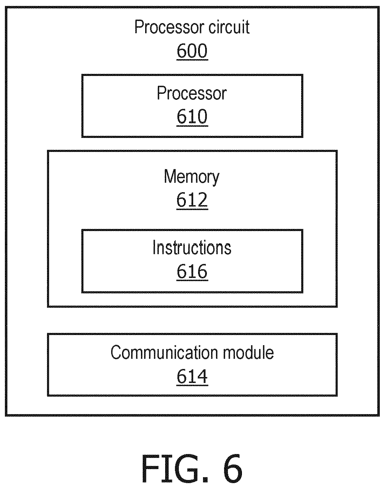

The processing system 510 is generally representative of any device suitable for performing the processing and analysis techniques disclosed herein. In some aspects, the processing system 510 includes a processor circuit, such as the processor circuit 600 of Fig. 6. In some aspects, the processing system 510 is programmed to execute steps associated with the data acquisition, analysis, and/or instrument (e.g., device) control described herein. Accordingly, it is understood that any steps related to data acquisition, data processing, instrument control, and/or other processing or control aspects of the present disclosure may be implemented by the processing system 510 (e.g., computing device) using corresponding instructions stored on or in a non-transitory computer readable medium accessible by the computing device. In some instances, the processing system 510 is a console device. Further, it is understood that in some instances the processing system 510 includes one or a plurality of computing devices, such as computers, with one or a plurality of processor circuits. In this regard, it is particularly understood that the different processing and/or control aspects of the present disclosure may be implemented separately or within predefined groupings using a plurality of computing devices. Any divisions and/or combinations of the processing and/or control aspects described below across multiple computing devices are within the scope of the present disclosure.

Fig. 6 is a schematic diagram of a processing system according to aspects of the present disclosure. The processor circuit 600 may be implemented in and/or as part of the processing system 510 of Fig. 5. As shown, the processor circuit 600 may include a processor 610, a memory 612, and a communication module 614. These elements may be in direct or indirect communication with each other, for example via one or more buses.

The processor 610 may include a central processing unit (CPU), a digital signal processor (DSP), an ASIC, a controller, an FPGA, another hardware device, a firmware device, or any combination thereof configured to perform the operations described herein. The processor 610 may also be implemented as a combination of computing devices, e.g., a combination of a DSP and a microprocessor, a plurality of microprocessors, one or more microprocessors in conjunction with a DSP core, or any other such configuration.

The memory 612 may include a cache memory (e.g., a cache memory of the processor 610), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and nonvolatile memory, or a combination of different types of memory. In some instances, the memory 612 includes a non-transitory computer-readable medium. The memory 612 may store instructions 616. The instructions 616 may include instructions that, when executed by the processor 610, cause the processor 610 to perform the operations described herein with reference to the processing system 510 (Fig. 5). Instructions 616 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements.

The communication module 614 may include any electronic circuitry and/or logic circuitry to facilitate direct or indirect communication of data between various components of the processor circuit 600 and/or the processing system 510 (Fig. 5). Additionally or alternatively, the communication module 614 may facilitate communication of data between the processor circuit 600, the display device 520, the input device 530, the cutting subsystem 540, the leaflet capture subsystem or balloon subsystem 550, the imaging device 560, the TAVR delivery catheter 580, and/or the like. In this regard, the communication module 614 may be an input/output (I/O) device interface, which may facilitate communicative coupling between the processor circuit 600 and (I/O) devices, such as the input device 530. Moreover, the communication module 614 may facilitate wireless and/or wired communication between various elements of the processor circuit 600 and/or the devices and systems of the system 500 using any suitable communication technology, such as a cable interface such as a USB, micro-USB, Lightning, or FireWire interface, Bluetooth, Wi-Fi, ZigBee, Li-Fi, or cellular data connections such as 2G/GSM, 3G/UMTS, 4G/LTE/WiMax, or 5G.

Turning back now to Fig. 5, the imaging device 560 may include an x-ray system, angiography system, fluoroscopy system, ultrasound system, computed tomography (CT) system, a magnetic resonance imaging (MRI) system, other suitable imaging devices, and/or combinations thereof. The imaging device 560 may additionally or alternatively include a nuclear medicine imaging device, such as a gamma camera or a single-photon emission computed tomography (SPECT) system, other suitable devices, and/or combinations thereof. In some aspects, the imaging device 560 may be configured to acquire imaging data of anatomy, such as the heart and blood vessels, while the imaging device 560 is positioned outside of the body of the patient. The imaging data may be visualized in the form of two-dimensional and/or three-dimensional images of the heart, blood vessel, and/or other anatomy. In some aspects, the imaging device 560 may be an internal device that is positioned inside the patient body. For example, the imaging device 560 may be an intracardiac echocardiography (ICE) catheter that obtains images while positioned within a heart chamber. In some aspects, the imaging device 560 may be an external device in that is it is positioned outside of the particular anatomy that is being imaged (e.g., blood vessels and/or heart), but is positioned inside the patient body. For example, the imaging device 560 may be a transesophageal echocardiography (TEE) probe that obtains images while positioned within an esophagus.

Moreover, the imaging device 560 may obtain images of the heart that are indicative of the health of the cardiac muscle or myocardium. In particular, the imaging device 560 may be configured to acquire imaging data that illustrates myocardial perfusion (e.g., myocardial perfusion imaging (MPI) data). For example, MPI data may be collected by imaging a radiopharmaceutical agent, such as thallium, in the patient's heart muscle using a SPECT system. Additionally or alternatively, the imaging data may be obtained by imaging one or more radiopaque markers embedded in body of an intraluminal device, for example, the outer/cutting sheath 546, the inner sheath 556, and/or the TAVR delivery catheter 580 to enable tracking and identify the location of the intraluminal device using fluoroscopy. In any case, the imaging data may illustrate vasculature and/or muscle mass with blood flow and/or vasculature and/or muscle mass that lack of blood flow in areas of the heart.

The balloon subsystem 550 may control pump 552 to capture the leaflets of the degenerated natural aortic valve or a degenerated replacement aortic valve. In some aspects, the human heart/heart cycle has two portions: systole and diastole. In that regard, the balloon catheter 554 is advanced through the aortic valve during systole when the heart muscle contracts and pumps blood from the chambers into the arteries. Then, during diastole when the heart muscle relaxes and the chambers of the heart are allowed to fill with blood, the leaflets of the aortic valve press against the outer surface of the balloon catheter 554. In some instances, during diastole, the inner sheath 556 is advanced such that tabs on the inner sheath 556 are positioned within the cusps of the aortic valve leaflets 304. The pump 552 of the balloon subsystem is then configured to expand the balloon on the balloon catheter 554 pinning the aortic valve leaflets 304 between an outer surface of the balloon and an inside surface of the inner sheath 556. In that regard, pump 552 is configured to transition the balloon between expanded state and the unexpanded state.

The cutting subsystem 540 may control laser source 542 and/or motor 544 for controlling a laser cutter and/or cutting tip disposed on a distal end of the outer/cutting sheath 546. In that regard, once the leaflets of the degenerated natural aortic valve or a degenerated replacement aortic valve are positioned between an outer surface of the balloon and an inside surface of the inner sheath 556, the outer/cutting sheath 546 may be advanced. In some aspects, the cutting subsystem 540 is configured to enable the laser source 542, so that one or more optical fibers disposed on a distal end of the outer/cutting sheath 546 may cut and/or resect the degenerated leaflets of the natural aortic valve or the replacement aortic valve. In some aspects, the cutting subsystem 540 is configured to enable the motor 544, so that one or more mechanical blades disposed on a distal end of the outer/cutting sheath 546 may cut and/or resect the degenerated leaflets of the natural aortic valve or the replacement aortic valve. Once the leaflets of the degenerated natural aortic valve or a degenerated replacement aortic valve, TAVR delivery catheter 580 delivers the TAVR valve 590 to the aortic valve area of the human heart, such that the TAVR valve 590 replaces either the degenerated natural aortic valve or a degenerated replacement aortic valve.

The system 500 includes a display device 520 that is communicatively coupled to the processing system 510. In some aspects, the display device 520 is a component of the processing system 510, while in other aspects, the display device 520 is distinct from the processing system 510. In some aspects, the display device 520 is a monitor integrated in a console device or a standalone monitor (e.g., a flat panel or flat screen monitor). The processing system 510 may be configured to generate a visual display (e.g., screen display) based on imaging data from an imaging device. The processing system 510 may provide (e.g., output) the screen display to the display device 520. To that end, the display device 520 may be configured to output (e.g., display) a two-dimensional image and/or a two-dimensional representation of the heart, blood vessels, and/or other anatomy, which may be included in the screen display. In some aspects, the display device 520 is configured to output a three-dimensional graphical representation of the heart, blood vessels, and/or other anatomy. For instance, the display device 520 may be a holographic display device configured to output a three-dimensional holographic display of anatomy. Any suitable display device is within the scope of this disclosure, including self-contained monitors, projection/screen systems, head-up display systems, etc. The display device may implement principles based on moving reflective microelectromechanical systems (MEMS), laser plasma, electro-holography, etc. In some aspects, the display device 520 is implemented as a bedside controller having a touch-screen display as described, for example, in U.S. Provisional Application No. 62/049,265, titled "Bedside Controller for Assessment of Vessels and Associated Devices, Systems, and Methods," and filed September 11, 2014, the entirety of which is hereby incorporated by reference herein.

The system 500 includes an input device 530 that is communicatively coupled to the processing system 510. The input device 530 may be a peripheral device, such as a touch sensitive pad, a touch-screen, a joy-stick, a keyboard, mouse, trackball, a microphone, an imaging device, and/or the like. In other aspects, the user interface device is part of the display device 520, which may be a touch-screen display, for example. Moreover, a user may provide an input to the processing system 510 via the input device 530. In particular, the input device 530 may enable a user to control, via inputs to the processing system 510, one or more of the components of the system 500, such as the cutting subsystem 540, the leaflet capture subsystem 550, the imaging device 560, the TAVR delivery catheter 580, or the processing system 510 itself. Additionally or alternatively, the input device 530 may facilitate interaction with a screen display provided at the display device 520. For instance, a user may select, edit, view, or interact with portions of the screen display (e.g., a GUI) provided at the display device 520 via the input device 530.

The system 500 may include various connectors, cables, interfaces, connections, etc., to communicate between the elements of the cutting subsystem 540, the leaflet capture subsystem 550, the imaging device 560, the TAVR delivery catheter 580, the processing system 510, the display device 520, and/or the input device 530. In some aspects, for example, the communication module 614 (Fig. 6), which may be included in the processing system 510, may include such connectors, interfaces, and/or the like. In this regard, the processing system 510 may communicate and/or control one or more components of the processing system 510 via mechanical and/or electromechanical signaling and/or controls. Further, the illustrated communication pathways are exemplary in nature and should not be considered limiting in any way. In this regard, it is understood that any communication pathway between the components of system 500 may be utilized, including physical connections (including electrical, optical, and/or fluid connections), wireless connections, and/or combinations thereof. In this regard, it is understood that the one or more of the components of the system 500 may communicate via a wireless connection in some instances. In some instances, the one or more components of the system 500 and/or other systems (e.g., of a hospital or health services provider) communicate via a communication link over a network (e.g., intranet, internet, telecommunications network, and/or other network).

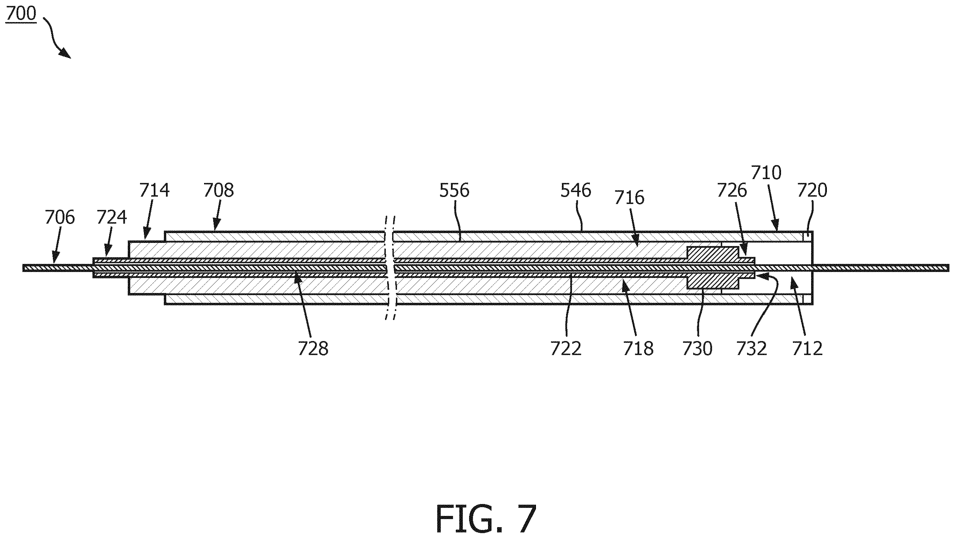

Fig. 7 is a diagrammatic cross-sectional top-view of an intraluminal device 700 according to aspects of the present disclosure. In the aspects of Fig. 7, visible are an outer/cutting sheath 546, an inner sheath 556, and guidewire 706. In some instances, the guidewire 706 is inserted through a body lumen such as an artery, e.g. the femoral artery, and advanced to the aorta 102 of the human heart 100. More specifically, the guidewire 706 is advanced to the aortic valve 124 region within the aorta 102. In some instances, the outer/cutting sheath 546 and/or the inner sheath 556 is placed over the guidewire 706 and advanced to the aortic valve 124 region. The outer/cutting sheath 546 includes a proximal end 708, a distal end 710, and a sheath lumen 712. Similarly, the inner sheath 556 includes a proximal end 714, a distal end 716, and a sheath lumen 718. In some instances, the distal end 716 of the inner sheath 556 is configured to be inserted into sheath lumen 712 at the proximal end 708 of the outer/cutting sheath 546. In that regard, the inner sheath 556 may be placed within the sheath lumen 712 of the outer/cutting sheath 546, the distal end 716 of the inner sheath 556 being placed over the guidewire 706 and the inner sheath 556 and the outer/cutting sheath 546 being placed within the body lumen and advanced together to the aortic valve 124 region. In some instances, the distal end 710 of the outer/cutting sheath 546 may be placed over the guidewire 706 in the sheath lumen 712, placed within the body lumen, and advanced to the aortic valve 124 region. In that regard, the distal end 716 of the inner sheath 556 may be inserted into sheath lumen 712 at the proximal end 708 of the outer/cutting sheath 546 and then advanced to the aortic valve 124 region. In some instances, the distal end 716 of the inner sheath 556 may be placed over the guidewire 706 in sheath lumen 718, placed within the body lumen, and advanced to the aortic valve 124 region. In that regard, the distal end 710 of the outer/cutting sheath 546 may be inserted over proximal end 714 of the inner sheath 556 and then advanced to the aortic valve 124 region. In some instances, the outer/cutting sheath 546 is a laser sheath. In that regard, the outer/cutting sheath 546 includes a cutting tip 720 disposed at a distal end 710.

In the aspects of Fig. 7, the intraluminal device 700 further includes a balloon catheter 722. The balloon catheter 722 includes a proximal end 724, a distal end 726, and a catheter lumen 728. The balloon catheter 722 may be inserted over the guidewire 706 and into the sheath lumen 718 at the proximal end 714 of the inner sheath 556 such that the guidewire 706 is positioned within the catheter lumen 728 of the balloon catheter 722. In some aspects, balloon 730 is disposed at the distal end 726 of the balloon catheter 722 spaced from and proximal to the distal tip 732. In some instances, the balloon 730 is formed of a polymer material. In some instances, an outer surface of the balloon 730 may be coated with a hydrophilic coating.

Aspects of the present disclosure may include features described in U.S. Provisional App. No. 63/415,724, filed October 13, 2022, and titled "Co-Registered Temporary Valve for Cardiac Valvular Interventions and Associated Devices, Systems, and Methods", and App. No. 63/443,819, filed on February 07, 2023, and titled "Concentric Sheath Assembly for Aortic Valve Leaflet Removal and Associated Devices, Systems, and Methods", the entireties of which are hereby incorporated by reference herein.

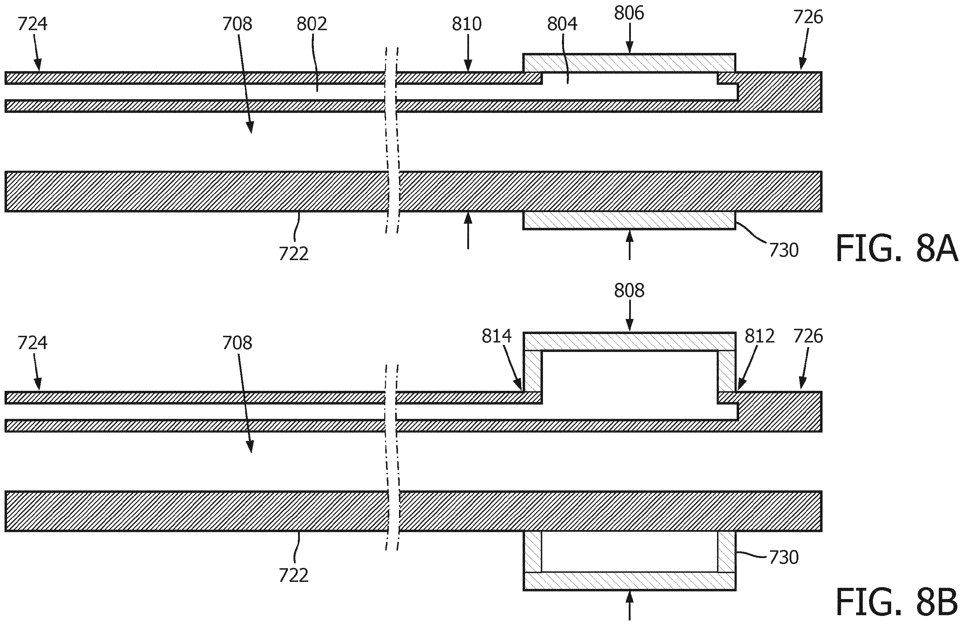

Figs. 8A and 8B illustrate a diagrammatic cross-sectional view of a balloon catheter 722 according to aspects of the present disclosure. In some instances, the balloon 730 includes an expandable structure (e.g., a plurality of ribs). The expandable structure may more generally have an unexpanded and/or deflated state (Fig. 8A) and an expanded and/or inflated state (Fig. 8B). In some instances, the balloon catheter 722 includes a balloon fluid lumen 802 in fluid communication with the pump 552 of the leaflet capture subsystem 550. In some instances, the pump 552 may be a fluid source. The balloon fluid lumen 802 is configured to receive fluid to transition the balloon 730 between the unexpanded state and the expanded state via a balloon fluid opening 804. In that regard, the balloon fluid lumen 802 includes a proximal end and a distal end. The proximal end of the balloon fluid lumen 802 is coupled to the pump 552 and the distal end of the balloon fluid lumen 802 includes the balloon fluid opening 804 that opens to an inside of the balloon 730 for expanding and deflating the balloon 730. In that regard, pump 552 is configured to transition the balloon 730 between expanded state and the unexpanded state. In some instances, a hemostasis valve may disposed on the proximal end of the balloon catheter 722. In the aspect of Fig. 8A, balloon 730 is shown in a deflated state. In the deflated state, the balloon 730 includes a deflated diameter 806 that is smaller than expanded diameter 808 but is larger than a first diameter 810 of the balloon catheter 722. In the aspect shown in Fig. 8B, in the expanded state, the balloon 730 includes an expanded diameter 808 that is larger than the first diameter 810 of the balloon catheter 722 and the deflated diameter 806. In some instances, the balloon 730 is coupled to the balloon catheter 722 at a leading edge 812 and at a trailing edge 814. In that regard, the leading edge 812 is coupled a distal end of the balloon fluid opening 804 and the trailing edge 814 is coupled at the proximal end of the balloon fluid opening 804.

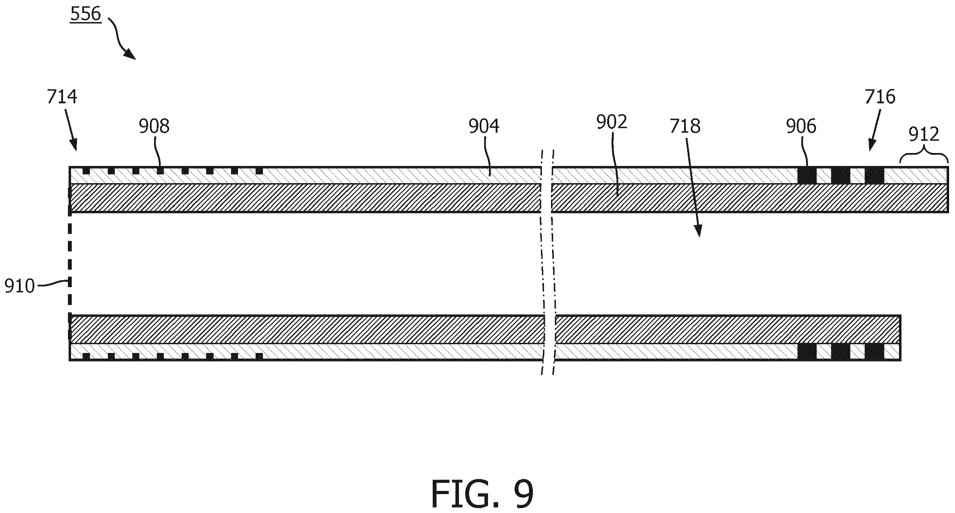

Fig. 9 is a diagrammatic cross-sectional top-view of an inner sheath 556 according to aspects of the present disclosure. In some instances, the inner sheath 556 includes a proximal end 714, a distal end 716, and a sheath lumen 718. In some instance, the inner sheath includes a perimeter. In some instances, an outer wall 902 of the inner sheath 556 may be coated with a hydrophilic coating 904. In some instances, disposed within the hydrophilic coating 904 on the distal end 716 of the inner sheath 556 are a set of radiopaque markers 906 to enable tracking and identify the location of the inner sheath 556 using fluoroscopy. In some instances, the set of radiopaque markers 906 may be spaced by a known distance. In some instances, disposed within the hydrophilic coating 904 on the proximal end 714 of the inner sheath 556 are a set of visible markers 908 to enable tracking and identify a depth of the inner sheath 556 by the human operator. In that regard, the set of visible markers 908 are optically visible, i.e. visible to the naked eye, because the proximal end 714 is positioned outside of the body of patient. In some instances, the set of visible markers 908 may be spaced by known distance. In some instances, a portion of the inner sheath 556 at the distal end 716 is elongated to form one or more tabs 912. As described hereafter, the one or more tabs 912 are utilized to capture the aortic valve leaflets 304 of Fig. 3A. In some instances, a hemostasis valve 910 may disposed on the proximal end of the sheath lumen 718. In that regard, the hemostasis valve 910 is designed to minimize fluid loss and backflow through the sheath lumen 718.

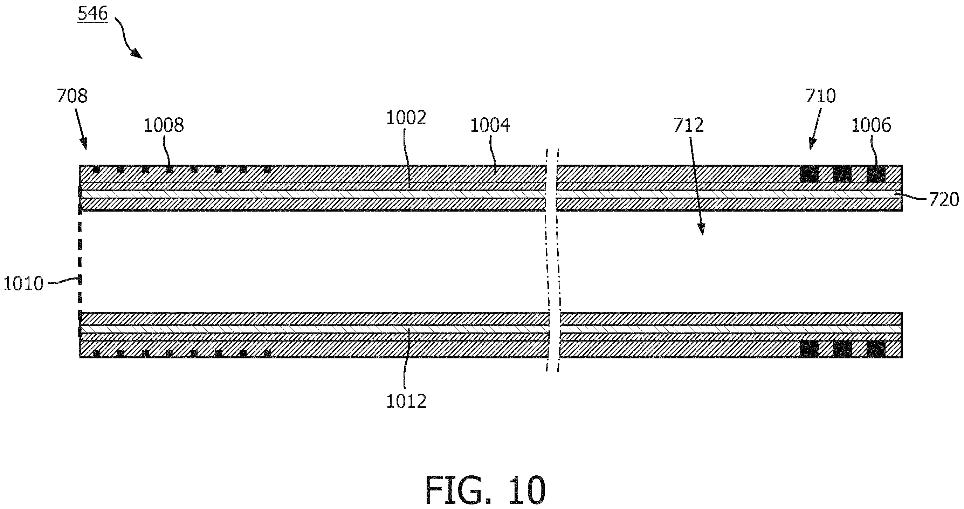

Fig. 10 is a diagrammatic cross-sectional top-view of an outer/cutting sheath 546 according to aspects of the present disclosure. In some aspects, the outer/cutting sheath 546 includes a proximal end 708, a distal end 710, and a sheath lumen 712. In some instances, an outer wall 1002 of the outer/cutting sheath 546 may be coated with a hydrophilic coating 1004. In some instances, disposed within the hydrophilic coating 1004 on the distal end 710 of the outer/cutting sheath 546 are a set of radiopaque markers 1006 to enable tracking and identify the location of the outer/cutting sheath 546 using fluoroscopy. In some instances, the set of radiopaque markers 1006 may be spaced by a known distance. In some instances, disposed within the hydrophilic coating 1004 on the proximal end 708 of the outer/cutting sheath 546 are a set of visible markers 1008 to enable tracking and identify a depth of the outer/cutting sheath 546 by the human operator. In that regard, the set of visible markers 1008 are optically visible, i.e. visible to the naked eye, because the proximal end 708 is positioned outside of the body of patient. In some instances, the set of visible markers 1008 may be spaced by known distance. In some instances, a hemostasis valve 1010 may disposed on the proximal end of the sheath lumen 712. In that regard, the hemostasis valve 1010 is designed to minimize fluid loss and backflow through the sheath lumen 712. In some instances, where the outer/cutting sheath 546 is a laser sheath, one or more optical fibers 1012 may traverse the length of the outer/cutting sheath 546. In that regard, a proximal end of the one or more optical fibers 1012 may be coupled to a laser source (laser source 542 of Fig. 5) such that the laser source emits a light the travels along the one or more optical fibers 1012 from the proximal end 708 to a distal end 710. In some aspects, the distal end of the one or more optical fibers 1012 may end at the distal end 710 of the outer/cutting sheath 546 forming cutting tip 720 such that the light emitted by the one or more optical fibers 1012 may cut and/or resect the degenerated leaflets of the natural aortic valve or the replacement aortic valve. Aspects of the outer/cutting sheath may include features similar to the GlideLight^{®} laser sheath available from Koninklijke Philips NV.

In other others, the cutting tip 720 can include blades that rotate to mechanically resect the leaflets. In such instances, the outer/cutting sheath 546 may include features similar to the TightRail^{®} mechanical rotation dilator sheath available from Koninklijke Philips NV.

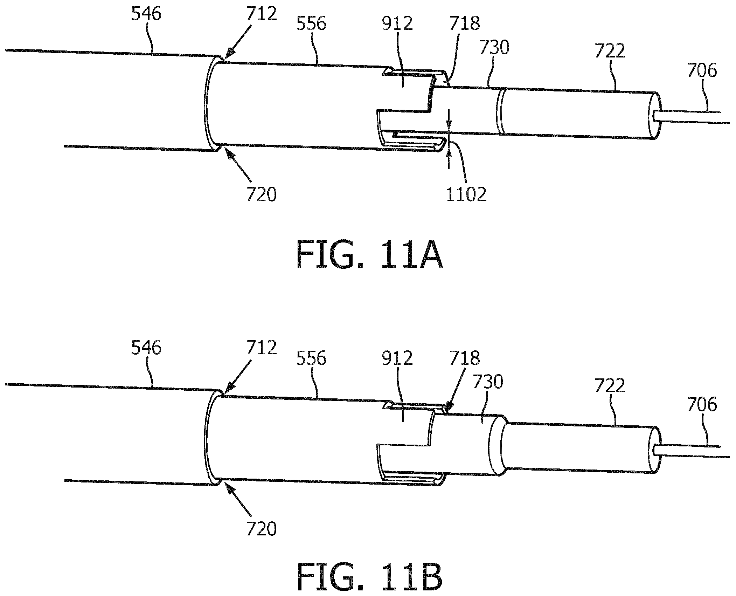

Figs. 11A and 11B are diagrammatic views of an intraluminal device 700 according to aspects of the present disclosure. Visible are the outer/cutting sheath 546, the inner sheath 556, the balloon catheter 722, and the guidewire 706. In some instances, the inner sheath 556 is disposed within the sheath lumen 712 of the outer/cutting sheath 546. In some instances, the balloon catheter 722 is disposed within the sheath lumen 718 of the inner sheath 556. In the aspects of Fig. 11A, an outside diameter of the balloon 730 in the deflated state disposed at the distal end of the balloon catheter 722 is smaller in diameter than an inside diameter of the inner sheath 556 forming a circumferential gap 1102. In some instances, the circumferential gap 1102, which is the difference in the outside diameter of the balloon 730 in the deflated state and the inside diameter of the inner sheath 556, is 1-5 millimeters. In some instances, the circumferential gap 1102, which is the difference in the outside diameter of the balloon 730 in the deflated state and the inside diameter of the inner sheath 556, is 2-5 millimeters. In some instances, the circumferential gap 1102, which is the difference in the outside diameter of the balloon 730 in the deflated state and the inside diameter of the inner sheath 556, is 2-3 millimeters. In that regard, the circumferential gap 1102 is sufficient in size for leaflets, such as the aortic valve leaflets 304 of Fig. 3A, to be drawn into the circumferential gap 1102 in order that a portion of the leaflets may be positioned between an inside of the tabs 912 of the inner sheath 556 and the balloon 730 so that the portion of the leaflets may be resected in anticipation of a new replacement TAVR valve. In some instances, a cutting tip 720 is disposed at the distal end of the outer sheath. In the aspects of Fig. 11B, when the balloon 730 is inflated, the balloon 730 expands, in some instances, to the inside diameter of the inner sheath 702. In other instances, when the leaflets are positioned between an outside of the balloon 730 and the inside of the tabs 912 of the inner sheath 556, the balloon 730 expands to a diameter to secure the leaflets between the outside of the balloon 730 and the inside of the tabs 912 of the inner sheath 556.

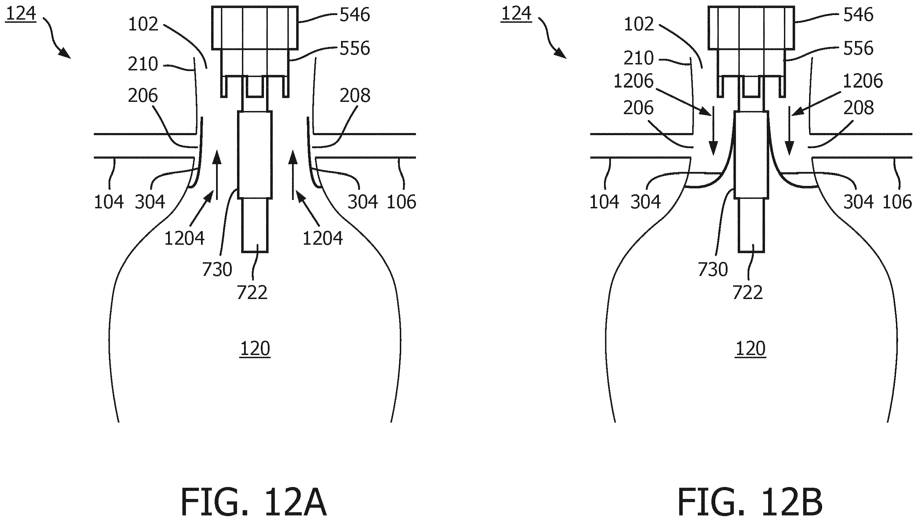

Figs. 12A and 12B are diagrammatic cross-sectional side views of an aortic valve 124 according to aspects of the present disclosure. Visible are the natural heart wall 210, the aortic valve leaflets 304, the right coronary artery 104, and the left main coronary artery 106. In the aspects of Fig. 12A, during systole indicated by directional arrows 1204, the aortic valve leaflets 304 open and allow blood to flow from the left ventricle 120 to the aorta 102 indicated by directional arrows 1204. In that regard, during systole, the balloon catheter 722 and the balloon 730, in the deflated state, are advanced between the aortic valve leaflets 304 and the aortic valve leaflets 304 are spaced from the balloon 730 and/or the balloon catheter 722. In the aspects of Fig. 12B, during diastole indicated by directional arrows 1206, the aortic valve leaflets 304 may be pushed by the flow of blood from the aorta 102 toward the left ventricle 120 such that the aortic valve leaflets 304 rest against and/or contact the balloon 730, in the deflated state, of the balloon catheter 722 to prevent mitral regurgitation.

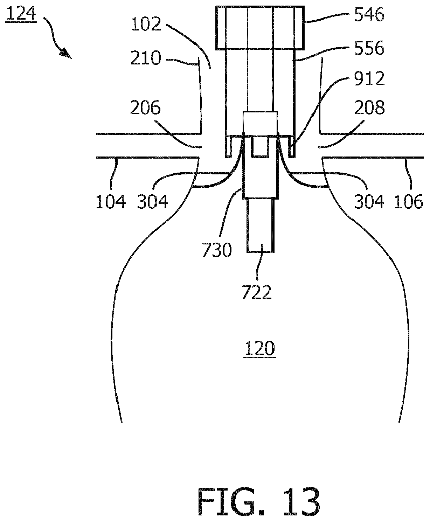

Fig. 13 is a diagrammatic cross-sectional side view of an aortic valve 124 according to aspects of the present disclosure. Fig. 13 includes features similar to those described in Figs. 12B. In the aspects of Fig. 13, during diastole, while the aortic valve leaflets 304 are resting against the balloon 730, in the deflated state of the balloon catheter 722, the inner sheath 556 is advanced, positioning the aortic valve leaflets 304 between an inside of the tabs 912 of the inner sheath 556 and an outside of the balloon 730 in the deflated state.

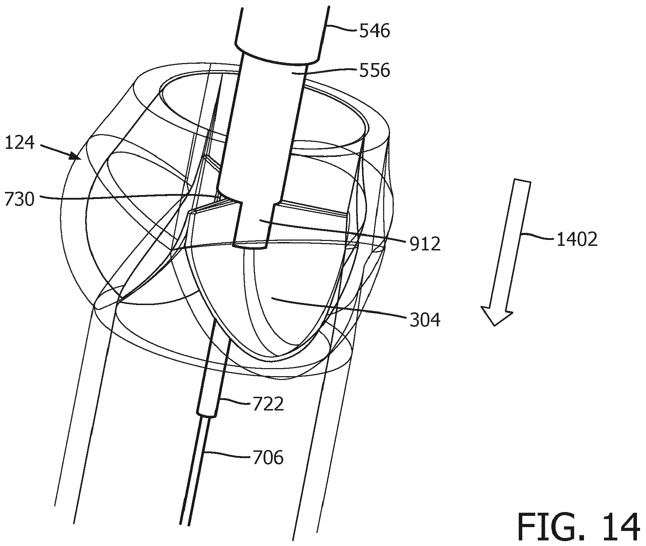

Fig. 14 is diagrammatic view of an intraluminal device 700 inserted into an aortic valve 124 of a human heart according to aspects of the present disclosure. Visible are the outer/cutting sheath 546, the inner sheath 556, the tabs 912, the balloon catheter 722, the balloon 730, and the guidewire 706. In the aspects of Fig. 14, the balloon catheter 722 has been advanced through the aortic valve 124 between the aortic valve leaflets 304 as indicated by directional arrow 1402. In some instances, during diastole, while the aortic valve leaflets 304 are resting against the balloon 730, in the deflated state, of the balloon catheter 722, the inner sheath 556 is advanced such that each one of the tabs 912 is positioned within a cusp and/or sinus of the aortic valve leaflets 304 thereby positioning each of the aortic valve leaflets 304 between a respective inside of the tabs 912 of the inner sheath 556 and an outside of the balloon 730, in the deflated state.

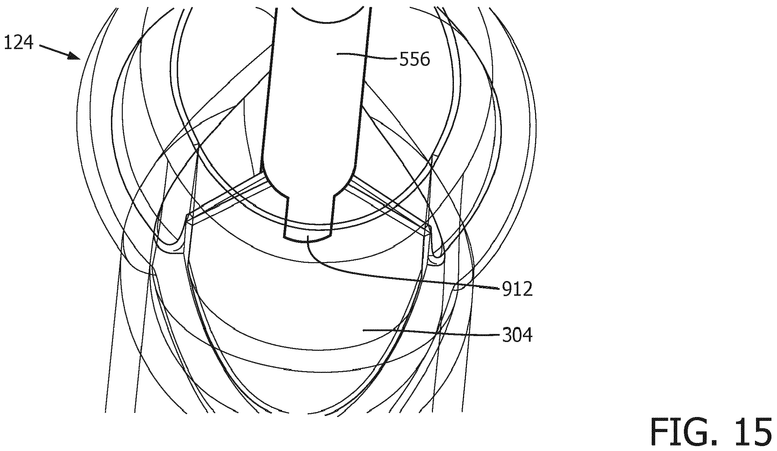

Fig. 15 is diagrammatic view of an inner sheath 556 positioned in an aortic valve 124 of a human heart according to aspects of the present disclosure. In the aspects of Fig. 15, one of the tabs 912 is positioned within a cusp of the aortic valve leaflets 304 thereby positioning the aortic valve leaflets 304 between the inside of the tabs 912 the inner sheath 556 and an outside of the balloon 730 (not visible) in the deflated state.

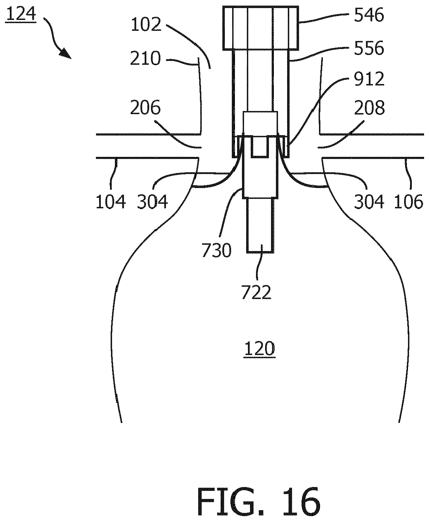

Fig. 16 is a diagrammatic cross-sectional side views of an aortic valve 124 according to aspects of the present disclosure. Fig. 16 includes features similar to those described in Figs. 12B and 13. In the aspects of Fig. 16, during diastole, while the aortic valve leaflets 304 are resting against the balloon 730 and the inner sheath 556 has been advanced, positioning the aortic valve leaflets 304 between an inside of the tabs 912 on the inner sheath 556 and an outer surface of the balloon 730, the balloon 730 is expanded to a larger diameter securing the aortic valve leaflets 304 between the outer surface of the balloon 730 and the inside of the tabs 912 of the inner sheath 556. In some instance, the outer surface of the balloon 730 is the outer surface of the balloon catheter 722.

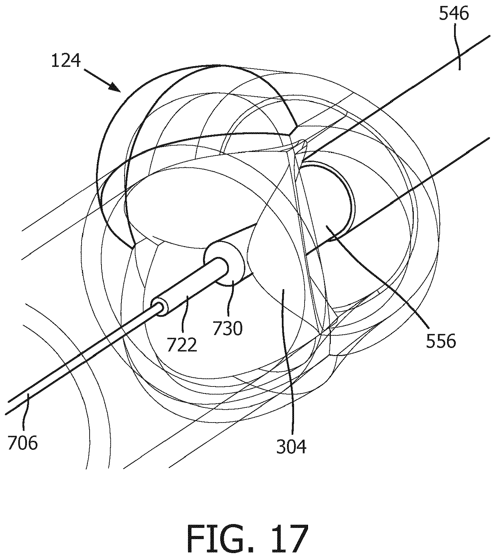

Fig. 17 is diagrammatic view of aortic valve leaflets 304 of an aortic valve 124 being secured between an outside of a balloon 730 in an inflated state and an inside of the inner sheath 556 according to aspects of the present disclosure. Visible are the outer/cutting sheath 546, the inner sheath 556, the tabs 912, the balloon catheter 722, the balloon 730, and the guidewire 706. In the aspects of Fig. 17, the balloon 730 is expanded to a larger diameter securing the aortic valve leaflets 304 between the outside of the balloon 730, in the expanded state, and the inside of the tabs 912 (not visible) of the inner sheath 556.

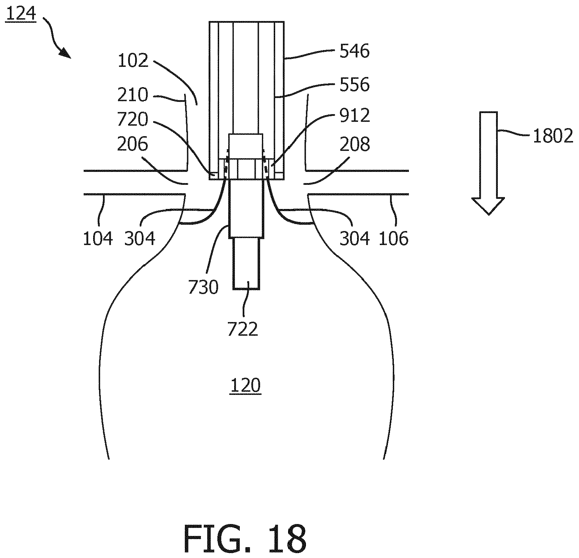

Fig. 18 is a diagrammatic cross-sectional side view of an aortic valve 124 according to aspects of the present disclosure. Visible are the natural heart wall 210, the aortic valve leaflets 304, the right coronary artery 104, and the left main coronary artery 106. In the aspects of Fig. 18, with the aortic valve leaflets 304 secured between the outside of the balloon 730, in the expanded state, and the inside of the tabs 912 of the inner sheath 556, the outer/cutting sheath 546 is advanced distally towards the distal portion of the inner sheath 556 as indicated by directional arrow 1802. In that regard, the cutting tip 720 at the distal end of the outer/cutting sheath 546 operates to resect a portion of the aortic valve leaflets 304 as the outer/cutting sheath 546 advances through the aortic valve leaflets 304.

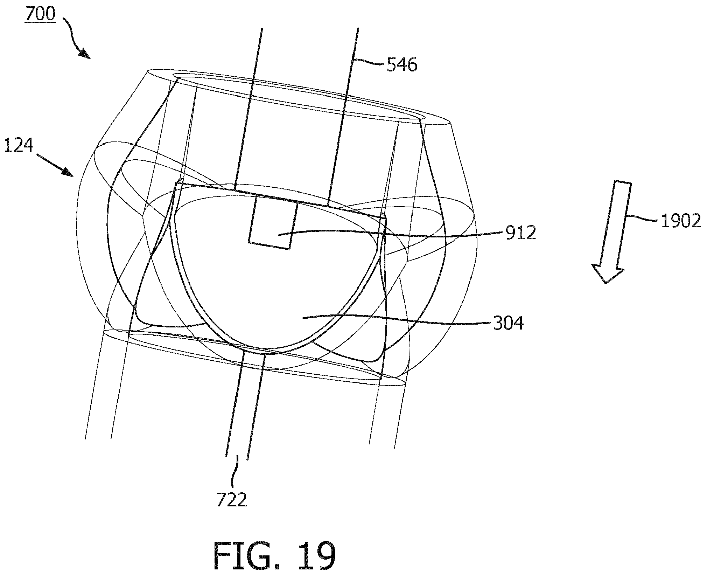

Fig. 19 is diagrammatic view of an intraluminal device 700 inserted into an aortic valve 124 of a human heart according to aspects of the present disclosure. Visible are the outer/cutting sheath 546, the tabs 912 at the end of the inner sheath 556, and the balloon catheter 722. In the aspects of Fig. 19, the outer/cutting sheath 546 has been advanced, as indicated by directional arrow 1902 to be in contact with the aortic valve leaflets 304 secured between the outside of the balloon 730 (not visible) and the inside of the tabs 912 of the inner sheath 556. In that regard, the cutting tip 720 at the distal end of the outer/cutting sheath 546 operates to resect a portion of the aortic valve leaflets 304 as the outer/cutting sheath 546 advances through the aortic valve leaflets 304.

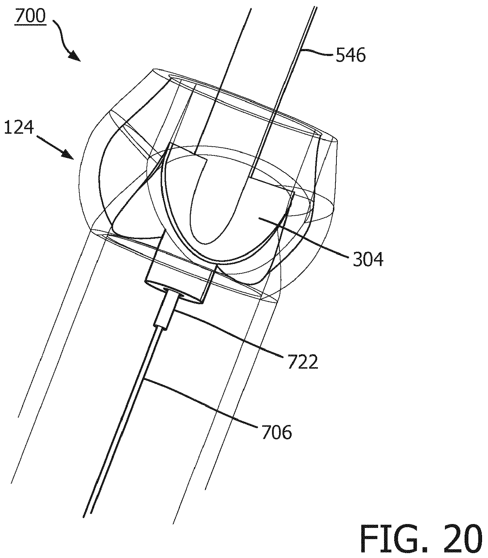

Fig. 20 is diagrammatic view of an intraluminal device 700 inserted into an aortic valve 124 of a human heart according to aspects of the present disclosure. Visible are the outer/cutting sheath 546, the balloon catheter 722, and the guidewire 706. In the aspects of Fig. 20, the outer/cutting sheath 546 has been advanced through the aortic valve 124 resecting portions of the aortic valve leaflets 304.

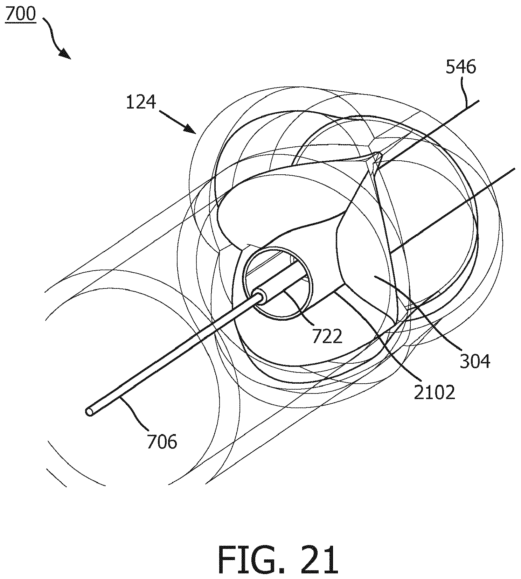

Fig. 21 is diagrammatic view of an intraluminal device 700 inserted into an aortic valve 124 of a human heart according to aspects of the present disclosure. Visible are the outer/cutting sheath 546, the balloon catheter 722, and the guidewire 706. In the aspects of Fig. 20, the outer/cutting sheath 546 has been advanced through the aortic valve 124 resecting portions of the aortic valve leaflets 304. In that regard, As shown in, e.g., Fig 21, the distal portion of the outer/cutting sheath 546 may be positioned distally past the distal portion of the inner sheath 556. In some instances, the resected portions 2102 of the aortic valve leaflets 304 remain captured between the outer surface of the balloon 730 (not visible) in the expanded state and the inside of the tabs 912 (not visible) of the inner sheath 556 (not visible). In some instances, movement of the inner sheath 556 alone (e.g., rotating the inner sheath 556 or translating the inner sheath 556), moves the cut portion of the leaflets along with the inner sheath 556. In some instances, movement of the inner sheath 556 and the balloon catheter 722 moves the cut portion of the leaflets along with the inner sheath 556 and the balloon catheter 722.

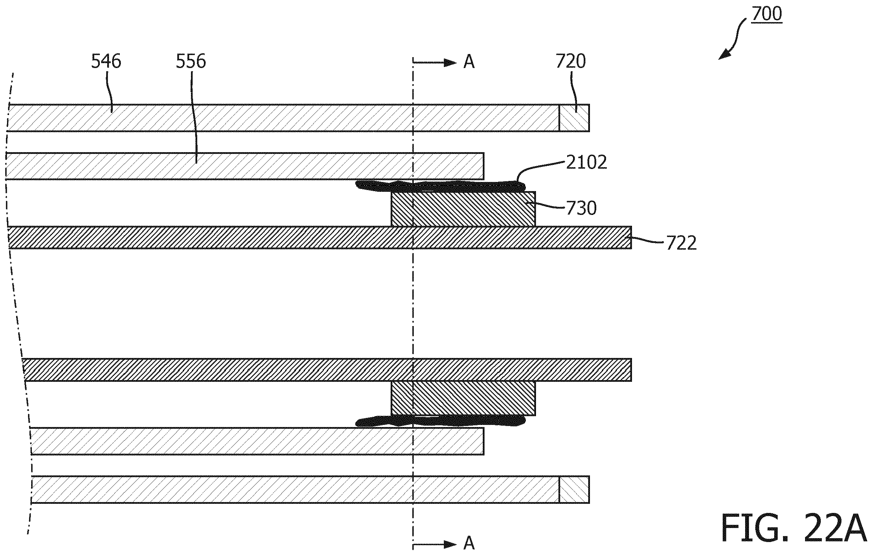

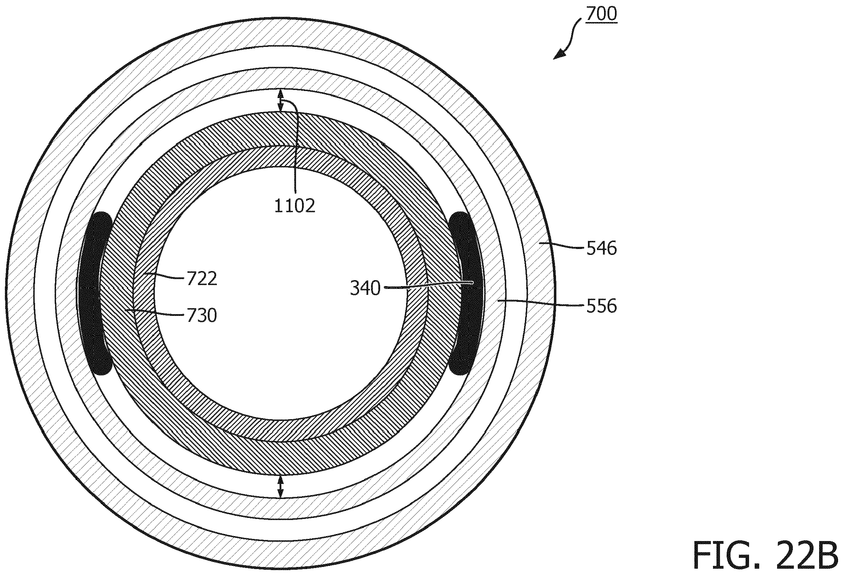

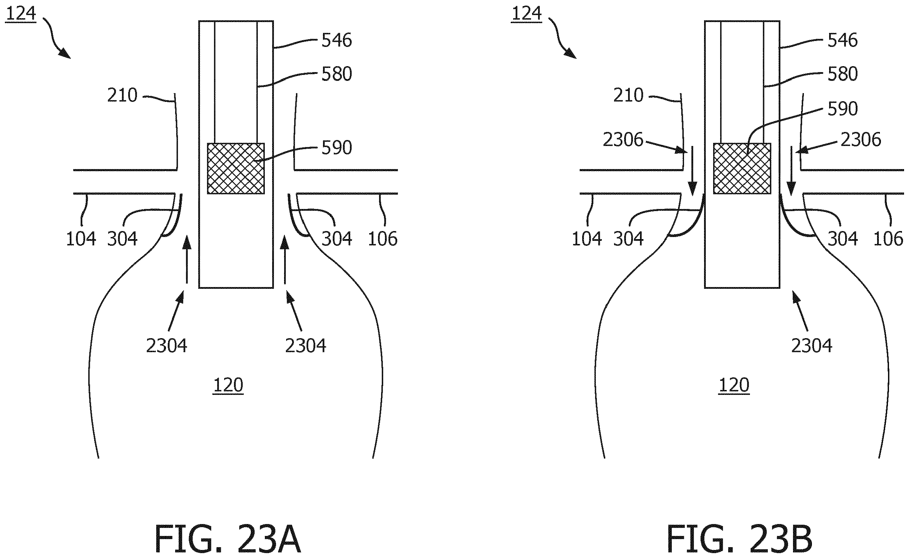

Fig. 22A is a diagrammatic cross-sectional top-view of an intraluminal device 700 according to aspects of the present disclosure. Visible are the outer/cutting sheath 546, the inner sheath 556, and the balloon catheter 722. In some instances, the cutting tip 720 is disposed at the distal end of the outer/cutting sheath 546 to resect portions of the aortic valve leaflets 304 once the aortic valve leaflets 304 are captured between an outer surface of the balloon 730, in the inflated state, and an inside wall of the inner sheath 556. In the aspects of Fig. 22A, the balloon 730, in the inflated state, secures the resected portions 2102 of the aortic valve leaflets 304 between an outer surface of the balloon 730 and inside wall of the inner sheath 556. Fig. 22B illustrates a cross-sectional view of the intraluminal device 700 as seen along the lines of the section A-A of Fig. 22A taken therein, according to aspects of the present disclosure. Visible are the outer/cutting sheath 546, the inner sheath 556, and the balloon catheter 722. In the aspects of Fig. 22B, the aortic valve leaflets 304 are pulled into the circumferential gap 1102 between an outer surface of the balloon 730, in the inflated state, and an inside wall of the inner sheath 556 capturing the aortic valve leaflets 304 in the circumferential gap 1102. To remove the resected portions 2102 from the patient body, the inner sheath 556 and the balloon catheter 722 can be removed from the patient body (e.g., with the balloon 730 inflated to hold the resected portions 2102 against the inside wall of the inner sheath 556) while the outer/cutting sheath 546 remains in place, as described with respect to Figs. 23A and 23B below.

Figs. 23A and 23B are diagrammatic cross-sectional side views of an aortic valve 124 according to aspects of the present disclosure. Visible are the natural heart wall 210, the aortic valve leaflets 304, the right coronary artery 104, and the left main coronary artery 106. In some aspects, after the aortic valve leaflets 304 have been resected by the advancement of the outer/cutting sheath 546 over the inner sheath 556, the inner sheath 556 is withdrawn from the outer/cutting sheath 546 taking the resected portions of the aortic valve leaflets 304 along with, so that the lumen of the outer/cutting sheath 546 is open. In the aspects of Fig. 23A, during systole indicated by directional arrows 2304, the aortic valve leaflets 304 open and allow blood to flow from the left ventricle 120 to the aorta 102 indicated by directional arrows 2304. In the aspects of Fig. 23B, during diastole indicated by directional arrows 2306, the aortic valve leaflets 304 may be pushed by the flow of blood from the aorta 102 toward the left ventricle 120 such that the aortic valve leaflets 304 rest against and/or contact the outer/cutting sheath 546 to prevent mitral regurgitation. In some aspects, the TAVR delivery catheter 580, along with the TAVR valve 590 disposed at the distal end of the TAVR delivery catheter 580, is inserted into the sheath lumen 712 of the outer/cutting sheath 546, positioned within the body lumen, and advanced to the aortic valve 124 region.

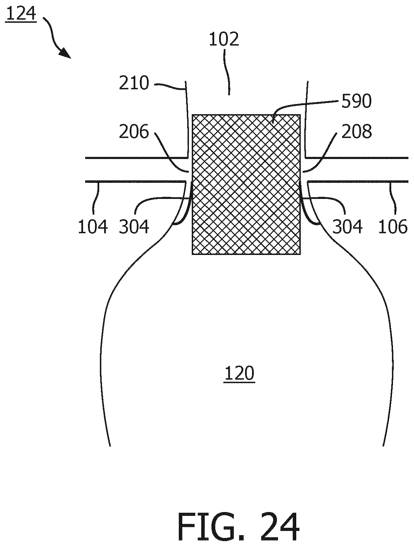

Fig. 24 is a diagrammatic cross-sectional side view of an aortic valve 124 according to aspects of the present disclosure. Visible are the natural heart wall 210, the aortic valve leaflets 304, the right coronary artery 104, and the left main coronary artery 106. In some aspects, the TAVR valve 590 is moved into position between the aorta 102 and the left ventricle 120. In some aspects, once positioned, the TAVR valve 590 is expanded pressing the resected aortic valve leaflets 304 against the natural heart wall 210. In that regard, the resected aortic valve leaflets 304 fail to obstruct the openings 206 and 208 to the right coronary artery 104 and the left main coronary artery 106, respectively.

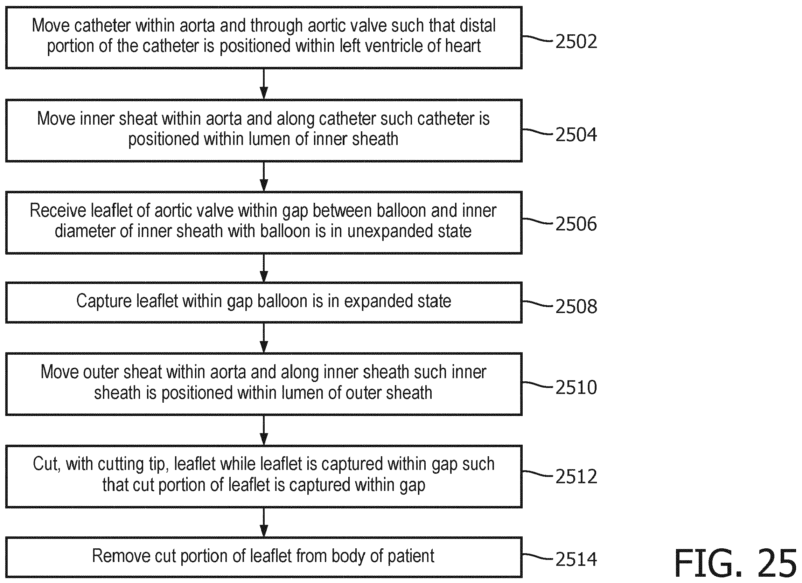

Fig. 25 is a flow diagram of the process performed in removing one or more portions of leaflets of a degenerated natural aortic valve or a degenerated replacement aortic valve according to aspects of the present disclosure. In step 2502, a catheter is moved within an aorta and through an aortic valve such that a distal portion of the catheter is positioned within a left ventricle of a heart. The catheter comprises a balloon positioned at the distal portion of the catheter and the balloon comprises an expanded state and an unexpanded state. Aspects of step 2502 are illustrated and described with respect to Figs. 12A and 12B. In step 2504, an inner sheath is moved within the aorta and along the catheter such the catheter is positioned within a lumen of the inner sheath. Aspects of step 2504 are illustrated and described with respect to Fig. 13. In step 2506, a leaflet of the aortic valve is received within a gap between the balloon and an inner diameter of the inner sheath with the balloon in the unexpanded state. In step 2508, the leaflet is captured within a gap with the balloon is in the expanded state. Aspects of step 2508 are illustrated and described with respect to Fig. 16. In step 2510, an outer sheath is moved within the aorta and along the inner sheath such the inner sheath is positioned within a lumen of the outer sheath. The outer sheath comprises a distal portion and a cutting tip positioned at the distal portion of the outer sheath. Aspects of step 2510 are illustrated and described with respect to Figs. 18, 19, 20, and 21. In step 2512, the leaflet is cut, with the cutting tip, while the leaflet is captured within the gap such that a cut portion of the leaflet is captured within the gap. Aspects of step 2512 are illustrated and described with respect to Figs. 22A and 22B. In step 2514, the cut portion of the leaflet is removed from a body of a patient. Aspects of step 2514 are illustrated and described with respect to Fig. 24.

Accordingly, it may be seen that the disclosed apparatus advantageously enables the replacement of an aortic valve in a non-obstructive way by removing old valve leaflets and preparing the implant site for a cleaner valve deployment and operation. By resection portions of the leaflets of the degenerated aortic valve, the replacement valve may be expanded without the current leaflets covering the openings to the coronary arteries, thereby allowing oxygenated blood to flow to the muscles of the heart.

The logical operations making up the aspects of the technology described herein are referred to variously as operations, steps, objects, elements, components, or modules. Furthermore, it should be understood that these may be arranged or performed in any order, unless explicitly claimed otherwise or a specific order is inherently necessitated by the claim language. It should further be understood that the described technology may be employed in single-use and multi-use electrical and electronic devices for medical or nonmedical use.

All directional references e.g., upper, lower, inner, outer, upward, downward, left, right, lateral, front, back, top, bottom, above, below, vertical, horizontal, clockwise, counterclockwise, proximal, and distal are only used for identification purposes to aid the reader's understanding of the claimed subject matter, and do not create limitations, particularly as to the position, orientation, or use of aspects of the present disclosure. Connection references, e.g., attached, coupled, connected, and joined are to be construed broadly and may include intermediate members between a collection of elements and relative movement between elements unless otherwise indicated. As such, connection references do not necessarily imply that two elements are directly connected and in fixed relation to each other. The term "or" shall be interpreted to mean "and/or" rather than "exclusive or." The word "comprising" does not exclude other elements or steps, and the indefinite article "a" or "an" does not exclude a plurality. Unless otherwise noted in the claims, stated values shall be interpreted as illustrative only and shall not be taken to be limiting.

The above specification, examples and data provide a complete description of the structure and use of exemplary aspects of the present disclosure, e.g., as defined in the claims. Although various aspects of the claimed subject matter have been described above with a certain degree of particularity, or with reference to one or more individual aspects, those skilled in the art could make numerous alterations to the disclosed aspects without departing from the spirit or scope of the claimed subject matter.

Still other aspects are contemplated. It is intended that all matter contained in the above description and shown in the accompanying drawings shall be interpreted as illustrative only of particular aspects and not limiting. Changes in detail or structure may be made without departing from the basic elements of the subject matter as defined in the following claims.

## Claims

1. A system, comprising:
an outer sheath configured to be positioned within a heart of a patient, wherein the outer sheath comprises a proximal portion, a distal portion, a lumen, and a cutting tip positioned at the distal portion;
an inner sheath configured to be positioned within the lumen of the outer sheath, wherein the inner sheath comprises a proximal portion, a distal portion and a lumen;
a catheter configured to be positioned within the lumen of the inner sheath, wherein the catheter comprises a proximal portion, a distal portion, and a balloon positioned at the distal portion, wherein the balloon is configured for an expanded state and an unexpanded state;
wherein, in the unexpanded state of the balloon, the catheter and the inner sheath are configured to receive a leaflet of an aortic valve within a gap between the balloon and an inner portion of the inner sheath;
wherein, in the expanded state of the balloon, the catheter and the inner sheath are configured to capture the leaflet within the gap; and
wherein the cutting tip of the outer sheath is configured to cut the leaflet while the leaflet is captured within the gap such that a cut portion of the leaflet is captured within the gap for removal from the patient.

2. The system of claim 1,
wherein the inner sheath comprises a plurality of tabs positioned spaced from one another around a perimeter at the distal portion of the inner sheath.

3. The system of claim 2, wherein the catheter and the inner sheath are configured to receive the leaflet and capture the leaflet between the balloon and at least one of the plurality of tabs.

4. The system of any of the preceding claims, further comprising a pump configured to transition the balloon between unexpanded state and the expanded state.

5. The system of any of the preceding claims, wherein the inner sheath comprises an outer surface with a hydrophilic coating.

6. The system of any of the preceding claims,
wherein at least one of the proximal portion of the inner sheath or the proximal portion of the outer sheath comprises a hemostasis valve.

7. The system of any of the preceding claims,
wherein at least one of the distal portion of the inner sheath or the distal portion of the outer sheath comprises a radiopaque marker.

8. The system of any of the preceding claims,
wherein at least one of the proximal portion of the inner sheath or the proximal portion of the outer sheath comprises a visible marker.

9. The system of any of the preceding claims, wherein the cutting tip comprises at least an optical fiber.

10. The system of any of the claims 1 to 9, wherein the cutting tip comprises rotatable blades.

11. A method, comprising:
moving a catheter within an aorta and through an aortic valve such that a distal portion of the catheter is positioned within a left ventricle of a heart, wherein the catheter comprises a balloon positioned at the distal portion of the catheter, wherein the balloon is configured for an expanded state and an unexpanded state;
moving an inner sheath within the aorta and along the catheter such that the catheter is positioned within a lumen of the inner sheath;
receiving a leaflet of the aortic valve within a gap between the balloon and an inner portion of the inner sheath with the balloon in the unexpanded state;
capturing the leaflet within a gap with the balloon in the expanded state;
moving an outer sheath within the aorta and along the inner sheath such the inner sheath is positioned within a lumen of the outer sheath, wherein the outer sheath comprises a distal portion and a cutting tip positioned at the distal portion of the outer sheath;
cutting, with the cutting tip, the leaflet while the leaflet is captured within the gap such that a cut portion of the leaflet is captured within the gap; and
removing the cut portion of the leaflet from a body of a patient.

12. The method of claim 11,
wherein the inner sheath comprises a proximal portion, a distal portion and a plurality of tabs positioned at the distal portion,
wherein the tabs are spaced from one another around the perimeter of the inner sheath,
wherein moving the inner sheath comprises positioning the plurality of tabs within sinuses of the aortic valve.

13. The method of claim 12, wherein the leaflet is received and captured between the balloon and at least one of the plurality of tabs.

14. The method of claim 11, wherein the distal portion of the catheter is positioned within the left ventricle such that:
the leaflet contacts the catheter during diastole to prevent mitral regurgitation; and
the leaflet is spaced from the catheter during systole to allow blood flow from the left ventricle to the aorta.

15. The method of claim 12, further comprising:
moving a valve delivery catheter through the lumen of the outer sheath, wherein the valve delivery catheter comprises a transcatheter aortic valve replacement valve removably coupled at a distal portion of the valve delivery catheter.
